# EUROPEAN PATENT SPECIFICATION

(11) **EP 3 801 889 B1**
(45) Date of publication and mention of the grant of the patent: **29.06.2022**
(21) Application number: 19748938.8
(22) Date of filing: 28.05.2019
(51) Int. Cl.: B01J 23/89, B01J 23/00, B01J 23/78, B01J 37/03, B01J 37/06, B01J 37/08, B01J 37/02, B01J 23/745, B01J 23/62, B01J 21/06, B01J 21/14, C07C 5/48, C07C 11/167, C07C 5/333

(54) **CATALYSTS FOR SINGLE STEP DOUBLE DEHYDROGENATION OF BUTADIENE FROM N-BUTANE**
KATALYSATOREN FÜR EINSTUFIGE DOPPELDEHYDRIERUNG VON BUTADIEN AUS N-BUTAN
CATALYSEURS POUR UNE DOUBLE DÉSHYDROGÉNATION, EN UNE SEULE ÉTAPE, DE BUTADIÈNE À PARTIR DE N-BUTANE

(30) Priority: 05.06.2018 US 201862680733 P
(43) Date of publication of application: 14.04.2021
(73) Proprietor: SABIC Global Technologies B.V., 4612 PX Bergen op Zoom (NL)
(72) Inventor: HAIDER, Muhammad H., Riyadh, 11551 (SA); AL-DAHLOUS, Waleed K., Riyadh, 11551 (SA); AHMED, Toseef, Riyadh, 11551 (SA)
(74) Representative: Patentanwälte Bressel und Partner mbB
(86) International application number: PCT/IB2019/054420
(87) International publication number: WO 2019/234553

(56) References cited:
- US-A- 5 219 816
- US-A1- 2010 240 941
- US-A1- 2015 202 601

## Description

### BACKGROUND OF THE INVENTION

### A. Field of the Invention

The invention generally concerns a catalyst for catalyzing double-dehydrogenation of butane. The catalyst can include gallium metal or oxide thereof and palladium deposited on an iron-magnesium-silicon oxide (FeMgSiO) support.

### B. Description of Related Art

Applications of butadiene (BD) in industries are increasing due to immense demand of BD as monomer for polymers, plastics, synthetic rubber or elastomers, noticeable of which are styrene butadiene rubber (SBR), polybutadiene rubber (PBR), polychloroprene (neoprene) and nitrile rubber (NR). Nearly 100% of BD produced globally is through steam cracking of ethylene, which obtain BD as a by-product from naphtha feedstock. Other competing technology for BD production is utilizing normal butenes feedstock, which can give reasonable yields. However, BD production from *n*-butane surpass both of the prelisted processes in terms of economics, as *n*-butane is an economical feed source.

Various attempts to use *n*-butane as a feed source for butadiene have been disclosed. By way of Example, U.S. Patent No. 7,488,858 to Johann et al. discloses producing butadiene from *n*-butane using multi-step processing using supported molybdenum and bismuth catalyst as the dehydrogenation catalyst used to dehydrogenate 1-butene to butadiene. In another example, U.S. Patent No. 9,193,647 to Giesa et al.*,* discloses producing butadiene from *n*-butane using multi-step processes that employs a Pt/Sn on alumina catalyst.

US 2015/202601 A1 discloses a FePtKGa/AlSi catalyst suitable for butadiene production.

In yet another example, European Patent Application No. EP2832716 to Haufe et al. describes using a 2 catalyst system that uses a first catalyst that includes platinum and Ge, Ga, In, or Sn, preferably Sn on a trimetallic magnesium aluminate, and at least one element from Column 14 and a second catalyst that includes oxides of Mg, Mo, Bi, Co, Ni, NiAl, Cu, Mn, Fe, and Ti on silica or zirconia. These processes suffer in that they require at least two catalysts and multiple processing steps and/or reactors.

While various supported promoted catalysts and processes to produce butadiene are known, these catalyst and/or processes involve multiple steps to produce butadiene; resulting in inefficient processes.

### SUMMARY OF THE INVENTION

A solution to some of the problems discussed above concerning conversion of *n-*butane to butadiene has been discovered. The solution is premised on support bimetallic catalyst that is capable of producing butadiene in a single-step process. The supported bimetallic catalyst includes a noble metal, a Column 13 or a Column 14 metal, on an iron-alkaline earth metal-silica based support. The iron or oxides thereof and alkaline earth metal or oxides thereof are dispersed throughout the silica matrix of the support and/or are arranged such that an iron core is surrounded by hierarchy of silicon and alkaline earth metal *(e.g.,* Mg) oxide. This is achieved by producing the silica support *in situ (e.g.,* through coprecipitation methods using tetra-alkyl silicate and iron citrate as a chelating agent). The catalytic metals comprising palladium (Pd) and gallium (Ga) can be deposited on the Fe-alkaline earth metal-silica support. Without wishing to be bound by theory, it is believed that when the iron is in the support material (*e.g*., in the core of the framework) it acts as a stabilizer for the *in situ* generated silica support for longer stability rather than a surface active metal.

In one aspect of the current invention, catalysts capable of producing butadiene from *n*-butane are described. A catalyst can include a catalytic Column 13 or Column 14 metal and a silica support that includes an alkaline earth metal or oxide thereof, and an iron metal or oxide thereof dispersed throughout the silica (Fe-alkaline earth metal-SiOₓ, where x balances the valence of the catalyst). The Fe-alkaline earth metal-SiOₓ supported catalyst includes a catalytic gallium and palladium metals oxides thereof, or alloys thereof. The alkaline earth metal can include magnesium, calcium, strontium, barium or mixtures thereof, preferably magnesium. In certain embodiments, the catalyst is absent a molybdenum or bismuth or compounds thereof, or combinations thereof. In some embodiments, the silica is not fumed silica. The Column 13 metal is Ga, the noble metal is Pd, and the Ga:Pd molar ratio is 0.01 to 0.5. The catalytic metals can be deposited on the Fe-alkaline earth metal-SiOₓ support. In one instance, the catalyst consists of gallium and palladium metal, oxides thereof, or alloys thereof on a FeMgSiO support. The catalyst of the present invention can be capable of catalyzing double dehydrogenation of butane with a selectivity of at least 20 mol.%, preferably 35 mol.%, more preferably at least 50 mol.%.

In another aspect of the invention, methods for preparing the catalyst of the present invention are described. A method can include the steps of: (a) obtaining a solution of a silicon precursor material (*e.g*., tetra-alkyl silicate such as tetraethyl orthosilicate (TEOS), an alkaline earth metal precursor material, and an iron chelated material (e.g., iron citrate); (b) adding an alkaline solution to the step (a) solution to precipitate a silica/alkaline-earth metal/iron material; (c) contacting the precipitated material with an oxidizing agent (*e.g.,* hydrogen peroxide (H₂O₂) to remove the chelating material (e.g., citrate); (d) heat treating (e.g., drying) the precipitating material to produce an Fe-alkaline earth metal-silica support material, where the iron and alkaline earth metal are dispersed throughout the silica; (e) contacting the Fe-alkaline earth metal-silica support material with a catalytic metal solution that include a gallium precursor under condition suitable to deposit gallium and then depositing palladium on the support material to form a bimetallic supported catalyst precursor material; and (f) heat treating the bimetallic supported catalyst precursor material under conditions suitable to from the catalyst of the present invention. The alkaline earth metal precursor material can include magnesium, calcium, strontium, barium, or combinations thereof, preferably a magnesium salt. Prior to step (c) the precipitated material can be dried at a temperature of 100 C to 150 °C, preferably 130 °C. Step (b) precipitation can include adding an alkaline solution comprising ammonia, preferably ammonium hydroxide to the solution. The oxidizing solution in step (c) can be hydrogen peroxide (H₂O₂). The step (b), (c) or step (f) material can be isolated and dried at 100 C to 150 °C, preferably 130 °C. The supported bimetallic material of step (f) can be calcined at a temperature of 300 °C to 550 °C, preferably 450 °C.

In yet another aspect of the present invention, methods of producing butadiene from *n*-butane are described. A method includes contacting a reactant feed that includes *n*-butane with the catalyst(s) of the present invention, under conditions sufficient to double dehydrogenate the butane and produce 1,3-butadiene. Conditions include temperatures from 450 °C to 600 °C, preferably, 500 °C to 600 °C, weighted hourly space velocity (WHSV) of 1000 h⁻¹ to 3000 h⁻¹, preferably 1200 h⁻¹ to 1500 h⁻¹, pressure of 0.001 MPa to 1 MPa, or combinations thereof. A *n*-butane volume to catalyst weight ratio is 100:1, preferably 50:1, 20:1, or 10:1, preferably 20:1. The butadiene selectivity of the catalyst can be at least 50 mol.%, at 550 °C to 575 °C. The butane conversion is at least 4 mol.%. In some embodiments, the reaction is done in the absence of hydrogen (H₂) and oxygen (O₂) gases.

The following includes definitions of various terms and phrases used throughout this specification.

An alkyl group is linear or branched, substituted or substituted, saturated hydrocarbon. Non-limiting examples of alkyl group substituents include alkyl, halogen, hydroxyl, alkyloxy, haloalkyl, haloalkoxy, carboxylic acid, ester, amine, amide, nitrile, acyl, thiol and thioether.

The terms "about" or "approximately" are defined as being close to as understood by one of ordinary skill in the art. In one non-limiting embodiment, the terms are defined to be within 10%, preferably within 5%, more preferably within 1%, and most preferably within 0.5%.

The terms "wt.%", "vol.%", or "mol.%" refers to a weight percentage of a component, a volume percentage of a component, or molar percentage of a component, respectively, based on the total weight, the total volume of material, or total moles, that includes the component. In a non-limiting example, 10 grams of component in 100 grams of the material is 10 wt.% of component.

The term "substantially" and its variations are defined to include ranges within 10%, within 5%, within 1%, or within 0.5%.

The terms "inhibiting" or "reducing" or "preventing" or "avoiding" or any variation of these terms, when used in the claims and/or the specification includes any measurable decrease or complete inhibition to achieve a desired result.

The term "effective," as that term is used in the specification and/or claims, means adequate to accomplish a desired, expected, or intended result.

The use of the words "a" or "an" when used in conjunction with any of the terms "comprising," "including," "containing," or "having" in the claims, or the specification, may mean "one," but it is also consistent with the meaning of "one or more," "at least one," and "one or more than one."

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The catalysts of the present invention can "comprise," "consist essentially of," or "consist of' particular ingredients, components, compositions, *etc.* disclosed throughout the specification. With respect to the transitional phrase "consisting essentially of," in one non-limiting aspect, a basic and novel characteristic of the catalysts of the present invention are their abilities to catalyze production of butadiene from *n*-butane in a single reactant unit.

In the context of the present invention, A catalyst capable of catalyzing double-dehydrogenation of butane is disclosed, the catalyst comprising a Column 13 or Column 14 metal or oxide thereof and a noble metal deposited on an iron-alkaline earth metal-silicon oxide support;
wherein the noble metal is palladium (Pd); wherein the Column 13 metal is gallium (Ga);
and wherein the Ga:Pd molar ratio is 0.01 to 0.5.

Preferably, the Column 13 metal or metal oxide is gallium oxide and palladium are deposited on an iron-magnesium-silicon oxide (FeMgSiO) support.

Preferably, the catalyst does not include molybdenum or bismuth. More preferably, the Ga:Pd molar ratio is 0.01 Preferably, the catalyst consists of gallium and palladium metal, oxides thereof, or alloys thereof on a FeMgSiO support.

The invention also relates to a method of producing butadiene, the method comprising contacting a feed stream comprising butane with the catalyst of the invention under conditions sufficient to double dehydrogenate the butane and produce a product stream that includes 1,3-butadiene; wherein the conditions comprise a temperature from 450 °C to 600 °C, a weighted hourly space velocity of 1000 h⁻¹ to 3000 h⁻¹ , a pressure of 0.1 to 1 MPa, wherein a *n*-butane volume to catalyst weight ratio is 100: 1.

Preferably, the temperature ranges from 500 °C to 600 °C. Preferably, the weighted hourly space velocity is 1500 h⁻¹ to 2000 h ⁻¹.

Preferably, the pressure is 0.1 MPa.

Preferably, a n-butane volume to catalyst weight ratio is 10:1.

More preferably, the reaction is done in the absence of hydrogen and oxygen gases.

The invention also relates to method of making the catalyst of of the invention, including the steps of obtaining a solution of a silicon precursor material, an alkaline earth metal precursor material and an iron precursor material; adding an alkaline solution to the step (a) solution to precipitate a silica/alkaline-earth metal/iron material; contacting the precipitated material with an oxidizing agent to remove the precursor material; heat treating the precipitating material to produce an Fe-alkaline earth metal-silica support material, wherein the iron and alkaline earth metal are dispersed throughout the silica; depositing a Column 13 or Column 14 metal precursor material on the Fe-alkaline earth metal-silica support material, and then depositing a noble metal precursor on the supported Column 13 or Column 14 material to form a supported bimetallic catalyst precursor material; and heat treating the supported bimetallic catalyst precursor material under conditions suitable to from the catalyst of the invention Preferably, the iron precursor material is iron citrate. Preferably, the method further comprises the step of isolating and drying the step (b), (c), or (f) precipitated material at a temperature of 100 °C to 150 °C, preferably 130 °C, and calcining the step (f) dried material at 300 °C to 550 °C, preferably 450 °C.

### DETAILED DESCRIPTION OF THE INVENTION

A discovery has been made that provides a solution to problems associated with catalysts used in processes to convert *n*-butane to butadiene. The discovery is premised on using a catalyst that includes catalytic metals and a silica support having iron or an oxide thereof and an alkaline earth metal or oxide thereof dispersed throughout the silica support. Non-limiting examples of the catalytic metals are noble metals *(e.g.,* Pd), Column 13 metals, Column 14 metals, and combinations thereof. According to the invention, Pd and Ga are used. Further, the catalytic activity and stability for the catalyst of the present invention is comparable or better as compared to the conventional catalysts for a multi-step process to produce butadiene. Therefore, the catalyst of the present invention provides a technical solution to at least some of the problems associated with the currently available catalysts for the dehydrogenation of butane mentioned above, such as low selectivity, low catalytic activity, and/or low stability.

These and other non-limiting aspects of the present invention are discussed in further detail in the following sections.

### A. Catalysts of the Present Invention

The catalyst of the present invention can be a supported catalyst with an ironstabilized alkaline earth metal-silica support. Non-limiting examples of alkaline earth metals (Column 2 of the Periodic Table) include Mg, Ca, Sr, Ba and combinations thereof. Non-limiting examples of catalytic metals (noble metals and Columns 13 and 14 of the Periodic Table include Rh, palladium (Pd), ruthenium (Ru), platinum (Pt), gold (Au), gallium (Ga), indium (In), germanium (Ge), antimony (Sb), and bismuth (Bi) oxides thereof, alloys thereof and mixtures thereof. The Fe-alkaline metal-silica support can include 0.5, 1, 1.5, 2, 2.5, to 3 wt.% or any value or range there between of iron and 20, 25, 30, 35, to 40 wt.% or any value or range there between of alkaline earth metal with the balance being silicon and oxygen. The catalyst of the present invention (Fe-alkaline metal-silica supported catalyst or physical mixture) can include up to 20 wt. % of the total amount of total catalytic transition metal, from 0.001 wt.% to 20 wt. %, from 0.01 wt. % to 15 wt. %, or from 1 wt. % to 10 wt. % and all wt.% or at least, equal to, or between any two of 0.001 wt.%, 0.01 wt.%, 0.1 wt.%, 1 wt.%, 2 wt.%, 3 wt.%, 4 wt.%, 5 wt.%, 6 wt.%, 7 wt.%, 8 wt.%, 9 wt.%, 10 wt.%, 15 wt.%, and 50 wt.%, with the balance support.

According to the invention, the catalyst includes gallium and palladium. The molar ratio of Ga:Pd in Fe-alkaline metal-silica supported catalyst or physical mixture is of 0.01 to 0.5 and all ranges and values there between including 0.01 to 0.5, 0.05 to 0.4, 0.01 to 0.30, or 0.01, 0.02, 0.03, 0.04, 0.05, 0.06, 0.07, 0.08, 0.09, 0.1, 0.2, 0.3, 0.4, 0.5 and all values and ranges there between. Overall, the active catalyst may have a composition of 3 to 20 wt.% palladium, 0.05 to 8 wt.% gallium, 40 to 80 wt.% silica, and 0.05 to 8 wt. % iron.

### B. Preparation of the Catalysts of the Present Invention

The Fe-alkaline metal-silica support of the present invention are made coprecipitation methodology. The method is such that the alkaline earth metal-silicates are first introduced into an aqueous media in the form of sol, which has certain dimensions in terms of water ligands, alkaline earth metal and silica portion. The iron chelated precursor, which acts as a chelating agent in the silica-alkaline earth metal sol, can then be added. The support material can be precipitating from solution using alkaline solution, washed and dried. The dried material can be washed with an oxidizing solution to remove the chelating agent *(e.g.,* citrate) and then dried. The catalytic metal can be precipitated or co-precipitated onto the dried support material. This methodology is in contrast to methods using hydrophilic silica (fumed silica) as a support. The resulting catalyst includes a bimetallic species decorated onto a porous support with iron core surrounded by hierarchy of silicon and alkaline earth metal *(e.g.,* Mg) oxide. In some embodiments, the iron is dispersed throughout the silicon and alkaline earth metal *(e.g.,* Mg) oxide porous support.

According to embodiments of the invention, a method may include providing an alkaline earth metal precursor solution. Non-limiting examples of the alkaline earth metal precursors may include magnesium chloride, magnesium acetate, calcium chloride, strontium chloride, strontium acetate, barium chloride, barium acetate, and combinations thereof. The solution can water. The alkaline earth metal salt solution may have a concentration in a range of 0.1 to 5 M and all ranges and values there between including 0.1 to 0.2 M, 0.2 to 0.4 M, 0.4 to 0.6 M, 0.6 to 0.8 M, 0.8 to 1.0 M, 1.0 to 1.2 M, 1.2 to 1.4 M, 1.4 to 1.6 M, 1.6 to 1.8 M, 1.8 to 2.0 M, 2.0 to 2.2 M, 2.2 to 2.4 M, 2.4 to 2.6 M, 2.6 to 2.8 M, 2.8 to 3.0 M, 3.0 to 3.2 M, 3.2 to 3.4 M, 3.4 to 3.6 M, 3.6 to 3.8 M, 3.8 to 4.0 M, 4.0 to 4.2 M, 4.2 to 4.4 M, 4.4 to 4.6 M, 4.6 to 4.8 M, and 4.8 to 5.0 M. In embodiments of the invention, the alkaline metal salt solution may be continuously stirred under a temperature in a range of 45 °C to 90 °C and all ranges and values there between. The duration for stirring may be in a range of 1 to 5 hours and all ranges and values there between.

A silica precursor material can be added to the alkaline earth metal solution. In some embodiments, the silica precursor material is added slowly (e.g., dropwise over time). Non-limiting examples of silica precursor material includes tetra-alkyl silicate, diethoxydimethylsilane (DEMS), tetramethyl orthosilicate (TMOS), tetraethyl orthosilicate (TEOS), and combinations thereof. In embodiments of the invention, the tetra-alkyl silicate can be TEOS. According to embodiments of the invention, the first mixture may have a alkaline earth metal to silicon weight (e.g., Mg:Si) ratio of 0.05 to 3 and all ranges and values there between including 0.05, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, and 3.0.

A precipitating agent can be added to the first mixture to form a second mixture. A non-limiting example of the precipitating agent may include ammonia or ammonia hydroxide *(e.g.,* 1 to 8 M, or 1, 2, 3, 4, 5, 6, 7, and 8 M). In embodiments of the invention, the amount of the precipitating agent added to the first mixture may be in a range of 45 to 100 mL, or and all ranges and values there between, including 45, 50, 55, 60, 65, 70, 75, 80, 85, 90, 95 and 100 mL. The second mixture may be continuously stirred for a duration of 0.5 to 5 hrs including 1, hrs, 2 hrs, 3 hrs, 4 hrs, and 5 hrs at a temperature of 20 to 30 °C, or about 25 °C until a gel is obtained. The composition of the second mixture can include at least, equal to or between any two of 20, 25, 30 and 40 wt.% magnesium, at least, equal to or between any two of 0.5, 1, 1.5, 2, 2.5 and 3 wt.% iron, at least, equal to or between any two of 57, 60, 65, 70, 75 to 79.5 wt.% silica.

The gel from the second mixture can be isolated (e.g., filtered or centrifuged), washed with hot water to remove the ammonia, and dried. Drying temperatures can range from 100 to 150 °C and all values and ranges there between including 100 to 105 °C, 105 to 110 °C, 110 to 115 °C, 115 to 120 °C, 120 to 125 °C, 125 to 130 °C, 130 to 135 °C, 135 to 140 °C, 140 to 145 °C, and 145 to 150 °C. The drying process may be 5 to 12 hrs and all ranges and values there between including 6 hrs, 7 hrs, 8 hrs, 9 hrs, 10 hrs, 11 hrs. The dried gel can be contacted with an oxidizing solution *(e.g.,* 50 mL of 10% H₂O₂). In some embodiments, contacting includes immersing the dried gel in an oxidizing solution. Contacting the dried gel with the oxidizing solution removes the remaining chelating material(s) and provides a solid material having iron and alkaline earth metal dispersed throughout the solid.

The dried Fe-alkaline earth metal-SiOₓ material can be precipitated or co-precipitated with the catalytic metal precursor (e.g., the noble metal and a Column 13 or Column 14 metal precursor). The dried Fe-alkaline earth metal-SiOₓ material can be dispersed in a solvent (e.g., water) and agitated at a temperature of 25 to 100 °C, 50 to 80 °C, or all values and ranges there between to form an aqueous dispersion. Agitation can range for 0.5 hours to 5 hours, or 1 to 3 hours or any values or ranges there between. A catalytic metal precursor solution can be added to the aqueous dispersion. One or more catalytic metal precursor solutions can be prepared by adding a catalytic metal salt *(e.g.,* a halide, nitrate, acetate, oxide, hydroxide, *etc.*) to a solvent. Non-limiting examples of the precursor solutions include an aqueous gallium oxide solution and an aqueous solution of a palladium nitrate.

The solutions can be added stepwise to the Fe-alkaline earth metal-Si support material or at the same time. In one example, the Column 13 or Column 14 metal precursor solution can be added to an aqueous solution of the support material of the present invention. The resulting dispersion can be agitated at for 0.5 hours to 15 hours, or 10 to 12 hours or any values or ranges there between at 25 to 110 °C, 80 to 100 °C, or all values and ranges there between to form solid material that includes the Column 13 or Column 14 metal on the support material. The supported material can be dried temperatures can range from 100 to 120 °C and all values and ranges there between including 100 to 105 °C, 105 to 110 °C, 110 to 115 °C, and 115 to 120 °C. The drying process may be 5 to 12 hrs and all ranges and values there between including 6 hrs, 7 hrs, 8 hrs, 9 hrs, 10 hrs, 11 hrs. The material can be reduced in size (e.g., powdered) and dispersed in water. The noble metal precursor solution can be added to Column 13 or 14 metal /support suspension. The resulting dispersion can be agitated at for 0.5 hours to 15 hours, or 10 to 12 hours or any values or ranges there between at 20 to 50 °C, 25 to 35 °C, or all values and ranges there between to form a supported bimetallic material. The supported bimetallic material can be dried temperatures can range from 100 to 150 °C and all values and ranges there between including 100 to 105 °C, 105 to 110 °C, 110 to 115 °C, 115 to 120 °C, 120 to 125 °C, 125 to 130 °C, 130 to 135 °C, 135 to 140 °C, 140 to 145 °C, and 145 to 150 °C. The drying process may be 5 to 12 hrs and all ranges and values there between including 6 hrs, 7 hrs, 8 hrs, 9 hrs, 10 hrs, 11 hrs.

The supported material of the present invention can be calcined at a temperature of 350 to 600 °C and all ranges and values there between including 350 to 360 °C, 360 to 370 °C, 370 to 380 °C, 380 to 390 °C, 390 to 400 °C, 400 to 410 °C, 410 to 420 °C, 420 to 430 °C, 430 to 440 °C, 440 to 450 °C, 450 to 460 °C, 460 to 470 °C, 470 to 480 °C, 480 to 490 °C, 490 to 500 °C, 500 to 510 °C, 510 to 520 °C, 520 to 530 °C, 530 to 540 °C, 540 to 550 °C, 550 to 560 °C, 560 to 570 °C, 570 to 580 °C, 580 to 590 °C, 590 to 600 °C to produce the catalytic bimetallic catalyst deposited on the Fe-alkaline earth metal silica support of the present invention. A heating rate for the calcination may be in a range of 1 to 5 °C/min and all ranges and values there between including 2 °C/min, 3 °C/min, and 4 °C/min. A calcination duration may be in a range of 2 to 12 hrs and all ranges and values there between including 3 hrs, 4 hrs, 5 hrs, 6 hrs, 7 hrs, 8 hrs, 9 hrs, 10 hrs, and 11 hrs.

The catalyst of the present invention can be further processed into a shaped form using known pelletizing, tableting procedures.

### C. Method of Producing 1,3-Butadiene from a Reactant Feed that Includes n-Butane.

The active bimetallic supported catalyst of the present invention can catalyze the conversion of a reactant feed that includes *n*-butane to produce a product stream that includes butadiene (*e.g*., 1,3-butadiene).

In embodiments of the invention, the feed stream can include 5 to 50 vol.% butane and the balance an inert gas (e.g., helium, nitrogen, argon, methane, and the like). The reaction conditions can include temperature, pressure and WHSV. The reaction temperature is in a range of 450 to 600 °C and preferably all ranges and values there between including 450 to 600 °C, 475 to 575 °C, and 500 to 550 °C. The reaction conditions include a reaction pressure in a range of 0 to 1.0 MPa and preferably all ranges and values there between including 0.2, 0.3, 0.4, 0.5, 0.6, 0.7, 0.8 and 0.9 MPa. In embodiments of the invention, a weight hourly space velocity for the synthesis gas is in a range of 1000 to 3000 hr⁻¹ and preferably all ranges and values there between 1200 to 1300 hr⁻¹, 1300 to 1400 hr⁻¹, 1400 to 1500 hr⁻¹, 1500 to 1600 hr⁻¹, 1600 to 1700 hr⁻¹, 1700 to 1800 hr⁻¹, 1800 to 1900 hr⁻¹, 1900 to 2000 hr⁻¹, 2000 to 2100 hr⁻¹, 2100 to 2200 hr⁻¹, 2200 to 2300 hr⁻¹, 2300 to 2400 hr⁻¹, and 2400 to 2500 hr⁻¹. Contact of the reactant gas feed with the catalyst produces a product stream that includes 1,3-butadiene olefins, and other by-products of the reaction. By-products such as other olefins and alkanes (e.g., olefins and alkanes having 2, 3, 4, and 5 carbon atoms (C2-C5)). Non-limiting examples of by-products include methane, ethane, ethylene, propylene, and butenes (*e.g*., trans-2-butene, 1-butene, *iso*-butene, cis-2-butene and the like). The by-products can be separated from the product stream using known separation methods such as distillation, membrane separations and the like, which are known in the art.

A conversion rate of the *n*-butane can be at least 4 to 100%, or at least, equal to, or between any two of 4%, 10%, 20%, 30%, 40%, 50%, 60%, 70%, 80%, 90%, and 100%. 1,3-butadiene selectivity can range from 50 to 100%, or at least, equal to, or between any two of 50, 55, 65, 70, 75, 80, 85, 90, 95 and 100%. In some instances, the 1,3-butadiene selectivity at 550 to 575 °C is at least 50% in a single pass.

The method can also include activating the catalyst prior to contact with the reactant feed. To activate the catalyst a gas stream including a reducing agent *(e.g.,* hydrogen) and a chemically inert gas *(e.g.,* nitrogen) can be contacted with the catalyst *(e.g.,* flow through the catalyst bed) at a temperature of 250 up to 450 °C and all ranges and values there between. A molar ratio for reducing gas to inert gas in the gas stream can be about 0.1:1. A heating rate for the activation may be in a range of 2 to 5 °C/min and all ranges and values there between including 3 °C /min and 4 °C /min. A weight hourly space velocity for the gas stream containing the reducing gas may be in a range of 3200 to 4000 hr⁻¹ and all ranges and values there between including 3200 to 3250 hr⁻¹, 3250 to 3300 hr⁻¹, 3300 to 3350 hr⁻¹, 3350 to 3400 hr⁻¹, 3400 to 3450 hr⁻¹, 3450 to 3500 hr⁻¹, 3500 to 3550 hr⁻¹, 3550 to 3600 hr⁻¹, 3600 to 3650 hr⁻¹, 3650 to 3700 hr⁻¹, 3700 to 3750 hr⁻¹, 3750 to 3800 hr⁻¹, 3800 to 3850 hr⁻¹, 3850 to 3900 hr⁻¹, 3900 to 3950 hr⁻¹, and 3950 to 4000 hr⁻¹.

An apparatus can be adapted for conversion of *n-*butane to 1,3-butadiene using the aforementioned active catalyst. The apparatus can include a fixed-bed flow reactor. The apparatus can include a catalyst bed in a fixed-bed flow reactor. The apparatus can also include a housing for containing the catalyst bed. In some embodiments the apparatus can include inlet means for introducing synthesis gas to the catalyst bed. The inlet means can an entrance adapted to receive *n*-butane. Further the apparatus can include an outlet means for removing the product stream that includes 1,3-butadiene from the apparatus. The outlet means can include an exit adapted to flow the product stream from the housing. The apparatus can include the catalyst according to embodiments of the invention disposed in the catalyst bed.

The apparatus may be a fluidized bed reactor, and/or a slurry reactor.

### EXAMPLES

The present invention will be described in greater detail by way of specific examples. The following examples are offered for illustrative purposes only, and are not intended to limit the invention in any manner.

### Materials Used

The following lab grade chemicals were obtained from SigmaMillipore without further purification magnesium chloride (1 M soln.), tetraethyl orthosilicate (TEOS), ferric citrate, liquid ammonia, hydrogen peroxide, palladium nitrate dihydrate (>97% purity), Cobalt (II) nitrate hexa hydrate (>98% purity).

### Example 1

### (Synthesis of GaPd/FeMgSiO support)

Magnesium chloride (20 ml of the 1 M solution) was diluted with deionized H₂O (to 100 mL) and stirred vigorously at 50 °C. Afterwards, TEOS (10.4 g) was dropped into it. Then, ferric citrate crystals (0.25 g) were added and continued to stir for 2 h. At this stage, NH₄OH (5 M, 36 ml stock up to 100 mL total) was added to the solution and the solution stirred for another 2 h before filtration to complete precipitation and then washed with hot water. The obtained material was dried overnight and then immersed in hydrogen peroxide (15% 50 ml) for 1 h. After drying the support material for 4 h, the dried support material (2.5 g) was suspended in ethanol (15 mL) and stirred for an hour at 25 to 35 °C (ambient temperature). Gallium oxide (0.35 g) was mixed with hydrochloric acid (10 g, 37%) for about 5 minutes and the added to the support suspension. The suspension was stirred for 10 to 15 hours at ambient temperature. The slurry was dried at about 100 °C for 5 h. The dried material was crushed to a powder form. The powder was suspended in water (15 mL). Palladium nitrate dihydrate (0.56 g) was dissolved in deionized water (15 mL) and added slowly to the aqueous support suspension. The resulting mixture was aged for 10 to 15 hours (overnight), followed by at 115 °C (5 h ramp at 1 °C/min) and calcination in static air at 450 °C (11 h, 5 °C/min). The catalyst was denoted by the symbol "A" hereafter.

### Example 2

### (Catalyst Activity/Selectivity Evaluation)

The catalyst from Example 1 was evaluated for the activity and selectivity for the production of 1,3-butadiene in a fixed bed flow reactor setup housed in temperature controlled system fitted with regulators to maintain pressure during the reaction. Prior to activity measurement, all of the catalysts were subjected to activation/reduction procedure which was performed at 250 °C (1h), 350 °C (1h) and 450 °C (1h) with the ramp rate of 3 °C min⁻¹ in 10:90 H₂/N₂ flow (WHSV: 1200 h⁻¹). The products of the reactions were analyzed through online GC analysis using a Varian GC (Agilent Scientific Instruments, U.S.A.) equipped with a TCD and FID detectors. The catalytic evaluation was carried out under the following conditions unless otherwise mentioned elsewhere; catalyst 0.5 g, temperatures 500 °C, 550 °C, 600 °C, WHSV 1200 h⁻¹, *n*-butane/helium ratio was 10/90, the feed flow was 100 mL min⁻¹, pressure of 0 to 1 bar (0 to 0.1 MPa) time on stream for each run was 2 h after 2 h of an initial stability. The mass balance of the reactions is calculated to be 100% ± 5.

Several catalysts are prepared and tested for the double dehydrogenation activity using the method of Example 1, and were found be selective for producing butadiene (BD) at a maximum of 50% from *n*-butane feed stock without utilizing hydrogen as a co-feed. Bimetallic gallium palladium supported on iron cored silica/magnesia porous support withstands the high temperature conditions required for double dehydrogenation and provides a good catalyst for on-purpose BD production from *n*-butane.

**Table 1**

| | | | |
|---|---|---|---|
| Temperature [°C] | 500 | 550 | 600 |
| Conversion (mol. %) | 4 | 5 | 7 |

| | **Selectivities (mol. %)** | | |
|---|---|---|---|
| 1,3-Butadiene | 36 | 50.32 | 22.12 |
| Methane | 0.14 | 0.96 | 3.97 |
| Ethane | 0.13 | 0.51 | 0.00 |
| Ethylene | 0.52 | 1.57 | 10.75 |
| Propane | 0.29 | 0.00 | 0.00 |
| Propylene | 0.57 | 2.20 | 11.93 |
| Trans-2-Butene | 35.66 | 20.64 | 30.71 |
| 1-Butene | 13.36 | 12.58 | 11.71 |
| Iso-butene | 0.65 | 0.85 | 0.00 |
| Cis-2-Butene | 12.69 | 10.42 | 8.81 |

## Claims

1. A catalyst capable of catalyzing double-dehydrogenation of butane, the catalyst comprising a Column 13 or Column 14 metal or oxide thereof and a noble metal deposited on an iron-alkaline earth metal-silicon oxide support;
wherein the noble metal is palladium (Pd);
wherein the Column 13 metal is gallium (Ga); and
wherein the Ga:Pd molar ratio is 0.01 to 0.5.

2. The catalyst of claim 1, wherein the Column 13 is gallium oxide and palladium are deposited on an iron-magnesium-silicon oxide (FeMgSiO) support.

3. The catalyst of any one of claim 1 to 2, wherein the catalyst does not include molybdenum or bismuth.

4. The catalyst of claim 1, wherein the Ga:Pd molar ratio is 0.01.

5. The catalyst of claim 1, wherein the catalyst consists of gallium and palladium metal, oxides thereof, or alloys thereof on a FeMgSiO support.

6. A method of producing butadiene, the method comprising contacting a feed stream comprising butane with the catalyst of any one of claims 1 to 3 under conditions sufficient to double dehydrogenate the butane and produce a product stream that includes 1,3-butadiene;
wherein the conditions comprise a temperature from 450 °C to 600 °C, a weighted hourly space velocity of 1000 h⁻¹ to 3000 h⁻¹, a pressure of 0.1 MPa to 1 MPa, wherein a *n-*butane volume to catalyst weight ratio is 100:1.

7. The method of claim 6, wherein the temperature ranges from 500 °C to 600 °C.

8. The method of any one of claims 6 to 7, wherein the weighted hourly space velocity is 1500 h⁻¹ to 2000 h⁻¹.

9. The method of any one of claims 6 to 7, wherein the pressure is 0.1 MPa.

10. The method of any one of claims 6 to 7, wherein a *n*-butane volume to catalyst weight ratio is 10:1.

11. The method of any one of claims 6 to 7, wherein that the reaction is done in the absence of hydrogen and oxygen gases.

12. A method of making the catalyst of any one of claims 1 to 2, the method comprising:
obtaining a solution of a silicon precursor material, an alkaline earth metal precursor material and an iron precursor material;
adding an alkaline solution to the step (a) solution to precipitate a silica/alkaline-earth metal/iron material;
contacting the precipitated material with an oxidizing agent to remove the precursor material;
heat treating the precipitating material to produce an Fe-alkaline earth metal-silica support material, wherein the iron and alkaline earth metal are dispersed throughout the silica;
depositing a Column 13 or Column 14 metal precursor material comprising gallium on the Fe-alkaline earth metal-silica support material, and then depositing a noble metal precursor comprising palladium such that the Ga:Pd molar ratio is 0.01 to 0.5. the supported Column 13 or Column 14 material to form a supported bimetallic catalyst precursor material; and
heat treating the supported bimetallic catalyst precursor material under conditions suitable to form the catalyst.

13. The method of claim 12, wherein the iron precursor material is iron citrate.

14. The method of any one of claims 12 to 13, further comprising isolating and drying the step (b), (c), or (f) precipitated material at a temperature of 100 °C to 150 °C, preferably 130 °C, and calcining the step (f) dried material at 300 °C to 550 °C, preferably 450 °C.

## Patentansprüche

1. Katalysator, der in der Lage ist, eine Doppel-Dehydrierung von Butan zu katalysieren, wobei der Katalysator ein Metall der Spalte 13 oder der Spalte 14 oder ein Oxid davon und ein Edelmetall umfasst, die auf einem Eisen-Erdalkalimetall-Siliciumoxid-Träger abgeschieden sind;
wobei das Edelmetall Palladium (Pd) ist;
wobei das Metall der Spalte 13 Gallium (Ga) ist; und
wobei das Ga:Pd-Molverhältnis 0,01 bis 0,5 ist.

2. Katalysator nach Anspruch 1, wobei das Spalte 13 Galliumoxid ist und Palladium auf einem Eisen-Magnesium-Siliciumoxid(FeMgSiO)-Träger abgeschieden sind.

3. Katalysator nach einem der Ansprüche 1 bis 2, wobei der Katalysator kein Molybdän oder Bismut enthält.

4. Katalysator nach Anspruch 1, wobei das Ga:Pd-Molverhältnis 0,01 beträgt.

5. Katalysator nach Anspruch 1, wobei der Katalysator von Gallium- und Palladiummetall, Oxiden davon oder Legierungen davon auf einem FeMgSiO-Träger gebildet wird.

6. Verfahren zum Produzieren von Butadien, wobei das Verfahren das Inkontaktbringen eines Feedstroms, der Butan umfasst, mit dem Katalysator nach einem der Ansprüche 1 bis 3 unter Bedingungen umfasst, die ausreichen, um das Butan doppelt zu dehydrieren und einen Produktstrom zu erzeugen, der 1,3-Butadien enthält;
wobei die Bedingungen eine Temperatur von 450 °C bis 600 °C, eine gewichtete stündliche Raumgeschwindigkeit von 1000 h⁻¹ bis 3000 h⁻¹, einen Druck von 0,1 MPa bis 1 MPa umfassen, wobei ein Verhältnis von n-Butan-Volumen zu Katalysatorgewicht 100:1 ist.

7. Verfahren nach Anspruch 6, wobei die Temperatur in einem Bereich von 500 °C bis 600 °C liegt.

8. Verfahren nach einem der Ansprüche 6 bis 7, wobei die gewichtete stündliche Raumgeschwindigkeit 1500 h⁻¹ bis 2000 h⁻¹ beträgt.

9. Verfahren nach einem der Ansprüche 6 bis 7, wobei der Druck 0,1 MPa beträgt.

10. Verfahren nach einem der Ansprüche 6 bis 7, wobei ein Verhältnis von n-Butan-Volumen zu Katalysatorgewicht 10:1 beträgt.

11. Verfahren nach einem der Ansprüche 6 bis 7, wobei die Umsetzung in Abwesenheit von Wasserstoff- und Sauerstoffgasen durchgeführt wird.

12. Verfahren zur Herstellung des Katalysators nach einem der Ansprüche 1 bis 2, wobei das Verfahren umfasst:
Beschaffen einer Lösung aus einem Silicium-Vorläufermaterial, einem Erdalkalimetall-Vorläufermaterial und einem Eisen-Vorläufermaterial;
Hinzufügen einer alkalischen Lösung zu der Lösung aus Schritt (a), um ein Siliciumdioxid/Erdalkalimetall/Eisen-Material zu fällen;
Inkontaktbringen des gefällten Materials mit einem Oxidationsmittel, um das Vorläufermaterial zu entfernen;
Wärmebehandeln des Fällungsmaterials, um ein Fe-Erdalkalimetall-Siliciumdioxid-Trägermaterial zu produzieren, wobei das Eisen und das Erdalkalimetall durch das Siliciumdioxid dispergiert sind;
Abscheiden eines Vorläufermaterials eines Metalls der Spalte 13 oder der Spalte 14, das Gallium umfasst, auf dem Fe-Erdalkalimetall-Siliciumdioxid-Trägermaterial und dann Abscheiden eines Edelmetall-Vorläufers, der Palladium umfasst, so dass das Ga:Pd-Molverhältnis 0,01 bis 0,5 beträgt, dem geträgerten Material der Spalte 13 oder der Spalte 14, um ein geträgertes bimetallisches KatalysatorVorläufermaterial zu bilden; und
Wärmebehandeln des geträgerten bimetallischen Katalysator-Vorläufermaterials unter Bedingungen, die geeignet sind, um den Katalysator zu bilden.

13. Verfahren nach Anspruch 12, wobei das Eisen-Vorläufermaterial Eisencitrat ist.

14. Verfahren nach einem der Ansprüche 12 bis 13, ferner ein Isolieren und Trocknen des gefällten Materials des Schrittes (b), (c) oder (f) bei einer Temperatur von 100 °C bis 150 °C und ein Kalzinieren des getrockneten Materials des Schrittes (f) bei 300 °C bis 550 °C, vorzugsweise 450 °C, umfassend.

## Revendications

1. Catalyseur capable de catalyser une double déshydrogénation du butane, le catalyseur comprenant un métal de la colonne 13 ou de la colonne 14 ou un oxyde de celui-ci et un métal noble déposé sur un support de fer-métal alcalinoterreux-oxyde de silicium ;
dans lequel le métal noble est du palladium (Pd) ;
dans lequel le métal de la colonne 13 est du gallium (Ga) ; et
dans lequel le rapport molaire Ga:Pd est 0,01 à 0,5.

2. Catalyseur selon la revendication 1, dans lequel la colonne 13 est un oxyde de gallium et du palladium qui sont déposés sur un support de fer-magnésium-oxyde de silicium (FeMgSiO).

3. Catalyseur selon la revendication 1 et 2, dans lequel le catalyseur n'inclut pas de molybdène ni de bismuth.

4. Catalyseur selon la revendication 1, dans lequel le rapport molaire Ga:Pd est 0,01.

5. Catalyseur selon la revendication 1, dans lequel le catalyseur se compose de gallium et de palladium métallique, d'oxydes de ceux-ci ou d'alliages de ceux-ci sur un support FeMgSiO.

6. Procédé de production de butadiène, le procédé comprenant la mise en contact d'un flux d'alimentation comprenant du butane avec le catalyseur selon l'une quelconque des revendications 1 à 3 dans des conditions suffisantes pour réaliser une double déshydrogénation du butane et produire un flux de produit qui inclut du 1,3-butadiène ;
dans lequel les conditions comprennent une température de 450 °C à 600 °C, une vitesse spatiale horaire pondérée de 1000 h⁻¹ à 3000 h⁻¹, une pression de 0,1 MPa à 1 MPa, dans lequel un rapport pondéral d'un volume de n-butane sur le catalyseur est 100:1.

7. Procédé selon la revendication 6, dans lequel la température est dans une plage de 500 °C à 600 °C.

8. Procédé selon la revendication 6 et 7, dans lequel la vitesse spatiale horaire pondérée est 1500 h⁻¹ à 2000 h⁻¹.

9. Procédé selon la revendication 6 et 7, dans lequel la pression est 0,1 MPa.

10. Procédé selon la revendication 6 et 7, dans lequel un rapport pondéral d'un volume de n-butane sur le catalyseur est 10:1.

11. Procédé selon la revendication 6 et 7, dans lequel la réaction est réalisée en l'absence d'hydrogène gazeux et d'oxygène gazeux.

12. Procédé de fabrication du catalyseur selon la revendication 1 et 2, le procédé comprenant :
l'obtention d'une solution d'un matériau précurseur de silicium, d'un matériau précurseur de métal alcalinoterreux et d'un matériau précurseur de fer ;
l'ajout d'une solution alcaline à la solution de l'étape (a) pour précipiter un matériau de silice/métal alcalinoterreux/fer;
la mise en contact du matériau précipité avec un agent oxydant pour éliminer le matériau précurseur ;
le traitement thermique du matériau de précipitation pour produire un matériau de support de fer-métal alcalinoterreux-silice, dans lequel le fer et le métal alcalinoterreux sont dispersés dans la silice ;
le dépôt d'un matériau précurseur de métal de la colonne 13 ou de la colonne 14 comprenant du gallium sur le matériau de support de fer-métal alcalinoterreux-silice, puis le dépôt d'un précurseur de métal noble comprenant du palladium, de sorte que le rapport molaire Ga:Pd soit 0,01 à 0,5, sur le matériau de la colonne 13 ou de la colonne 14 supporté pour former un matériau précurseur de catalyseur bimétallique supporté ; et
le traitement thermique du matériau précurseur de catalyseur bimétallique supporté dans des conditions appropriées pour former le catalyseur.

13. Procédé selon la revendication 12, dans lequel le matériau précurseur de fer est du citrate de fer.

14. Procédé selon la revendication 12 et 13, comprenant en outre l'isolation et le séchage du matériau précipité à l'étape (b), (c), ou (f) à une température de 100 °C à 150 °C, de préférence 130 °C, et la calcination du matériau séché à l'étape (f) à une température de 300 °C à 550 °C, de préférence 450 °C.
